Europäisches Patentamt

European Patent Office

Office européen des brevets

(19)

(11) Veröffentlichungsnummer: **0 002 808**
**B1**

(12) **EUROPÄISCHE PATENTSCHRIFT**

(21) Anmeldenummer: **78101802.3**

(22) Anmeldetag: **21.12.78**

(51) Int. Cl.³: **C 07 C 53/40, C 07 C 51/42**

(54) Verfahren zur Gewinnung reinen Acetylchlorids

(30) Priorität: **29.12.77 DE 2758682**

(43) Veröffentlichungstag der Anmeldung:
**11.07.79 Patentblatt 79/14**

(45) Bekanntmachung des Hinweises auf die Patenterteilung:
**28.01.81 Patentblatt 81/4**

(84) Benannte Vertragsstaaten:
**BE DE FR GB NL**

(56) Entgegenhaltungen:
**GB - A - 397 775**
**US - A - 2 748 151**

(73) Patentinhaber: **HOECHST AKTIENGESELLSCHAFT**
**Postfach 80 03 20**
**D-6230 Frankfurt/Main 80 (DE)**

(72) Erfinder: **Ohorodnik, Alexander, Dr.**
**Kastanienweg 24**
**D-5042 Erftstadt (DE)**
(72) Erfinder: **Auer, Eberhard, Dr.**
**Berliner Strasse 1**
**D-5042 Erftstadt (DE)**
(72) Erfinder: **Gehrmann, Klaus, Dr.**
**Geschwister Scholl-Strasse 32**
**D-5042 Erftstadt (DE)**

Verfahren zur Gewinnung reinen Acetylchlorids

Acetylchlorid ist ein in der chemischen Synthese vielseitig verwendbares Reagenz. Sein Einsatzgebiet und sein Preis sind von seiner Reinheit abhängig. Technisches Acetylchlorid ist etwa 98%ig, während für viele Anwendungszwecke ein mindestens 99%iges Acetylchlorid gefordert wird.

Bei der Herstellung von 2,5-Dioxo-1-oxa-2-phospholanen aus Dichlorphosphanen, Acrylsäure und Essigsäureanhydrid fallen große Mengen verunreinigtes Acetylchlorid an, die aus Gründen des Umweltschutzes und der Wirtschaftlichkeit nicht einfach durch Hydrolyse und Neutralisation oder durch Verbrennung vernichtet werden können. Es bestand daher die — Aufgabe, das rohe Acetylchlorid in eine möglichst reine und damit vielseitig verwendbare Form zu überführen.

Das zu reinigende Acetylchlorid kann aufgrund seiner Entstehungsweise bis zu etwa 10 Gew% Verunreinigungen enthalten, die an sich auf bekannte Weise, z.B. durch fraktionierte Destillation, abgetrennt werden können. Lediglich der Gehalt an gebundenem Phosphor, der Schwankungen unterworfen ist und bis zu 1000 ppm betragen kann, läßt sich auf diese Weise nicht ausreichend vermindern. Für viele Anwendungen des Acetylchlorids ist es hingegen erwünscht und notwendig, daß sein P-Gehalt 1 ppm nicht übersteigt. Es bestand somit die Aufgabe, das Acetylchlorid zu reinigen und seinen P-Gehalt auf unter 1 ppm zu senken.

Analytische Untersuchungen haben gezeigt, daß der P-Gehalt auf einer Verunreinigung durch Phosphortrichlorid beruht, das bei der Herstellung von 2,5-Dioxo-1-oxa-2-phospholanen als Verunreinigung des Dichlorphosphans eingeschleppt wird. Weitere Untersuchungen haben ergeben, daß eine einfache destillative Abrennung kleiner Mengen $PCl_3$ (Kp. 75,9°C) aus Acetylchlorid (Kp. 50,9°C) nicht möglich ist.

Die Siede- und Taukurven des Systems $PCl_3$/Acetylchlorid liegen in diesem Konzentrationsbereich derart eng beieinander, daß eine Trennung nur in einer Kolonne mit einer unwirtschaftlichen hohen Bodenzahl erfolgen könnte.

Die schwierige Aufgabe konnte überraschenderweise dadurch gelöst werden, daß das Acetylchlorid zunächst einer Behandlung mit Chlor unterzogen und anschließend fraktioniert destilliert wird. Bei der Chlorierung wird das $PCl_3$ in $PCl_5$ überführt, welches ein normales Siedeverhalten (Subl.p. 159°C) im Gemisch mit Acetylchlorid zeigt.

Das bei dem erfindungsgemäßen Reinigungsverfahren anfallende Acetylchlorid ist über 99%ig und enthält weniger als 1 ppm Phosphor.

Im einzelnen betrifft die Erfindung nunmehr ein Verfahren zur Gewinnung reinen Acetylchlorids mit einem Phosphorgehalt unter 1 ppm, welches dadurch gekennzeichnet ist, daß man das rohe, Phosphorverbindungen enthaltende, Acetylchlorid in innige Berührung mit 1 bis 20, vorzugsweise 10 bis 15, Liter gasförmigem Chlor je Liter flüssiges, rohes Acetylchlorid bringt und anschließend in an sich bekannter Weise fraktioniert destilliert.

### Beispiel 1
(Vergleichsbeispiel)

Eine Destillationsapparatur, bestehend aus einem Umlaufverdampfer von 2,5 l Inhalt, einer mit 8 mm-Sattelkörpern gefüllten Kolonne von 2 m Länge und 50 mm lichter Weite und einem Kühler mit Rücklaufteiler, wird stündlich mit 2,8 l Roh-Acetylchlorid folgender Zusammensetzung beschickt (Gewichts-%):

| | |
|---|---|
| Acetylchlorid | 85,97 |
| Essigsäure | 0,52 |
| Acrylsäurechlorid | 4,94 |
| Vinylidenchlorid ($CH_2 = CCl_2$) | 0,12 |
| Essigsäureanhyrid | 7,60 |
| gemischtes Essigsäure-Acrylsäureanhydrid | 0,86 |
| P-Gehalt | 125 ppm |

Bei einer Temperatur des Umlaufverdampfers von 82—83°C und einem Rücklaufverhältnis von 1:1 werden stündlich 2,32 l Rein-Acetylchlorid folgender Zusammensetzung über Kopf abgenommen (Gewichts-%):

| | |
|---|---|
| Acetylchlorid | 99,32 |
| Acrylsäurechlorid | 0,52 |
| Essigsäureanhydrid | 0,15 |
| P-Gehalt | 9 ppm |

Es werden stündlich 475 ml Sumpf aus dem Umlaufverdampfer ausgeschleust. Der Versuch dauerte 24 Stunden.

### Beispiel 2

In ein senkrecht stehendes Rohr von 100 mm lichter Weite und 120 cm Länge, das mit groben Sattelkörpern gefüllt ist, werden von oben stündlich 2,4 l Roh-Acetylchlorid der in Beispiel 1 angegebenen Zusammensetzung eingebracht, während von unten stündlich 29—30 l Chlorgas eingeblasen werden. Das chlorhaltige Acetylchlorid wird über ein Steigrohr abgenommen der in Beispiel 1 beschriebenen Destillationsapparatur zugeführt. Bei einer Temperatur des Umlaufverdampfers von 83—84°C und einem Rücklaufverhältnis von 1:1 werden stündlich 1,96 l Rein-Acetylchlorid folgender Zusammensetzung abgenommen (Gewichts-%):

| | |
|---|---|
| Acetylchlorid | 99,78 |
| Essigsäureanhydrid | 0,22 |
| P-Gehalt | unter 1 ppm |

Der Versuch lief 36 Stunden. Aus dem Umlaufverdampfer wurden insgesamt 15,5 l Sumpf ausgeschleust.

### Beispiel 3

In der in Beispiel 2 beschriebenen Apparatur werden stündlich 2,2 l Roh-Acetylchlorid folgender Zusammensetzung in Gewichts%

| | |
|---|---|
| Acetylchlorid | 86,31 |
| Essigsäure | 0,59 |
| Acrylsäurechlorid | 4,94 |
| Vinylidenchlorid | 0,06 |
| Essigsäureanhydrid | 7,15 |
| gemischtes Essigsäure Acrylsäureanhydrid | 0,95 |
| p-Gehalt | 26 ppm |

mit 25—26 l/h Chlorgas behandelt und anschließend in der beschriebenen Apparatur destilliert. Bei einer Temperatur des Umlaufverdampfers von 83—84°C und einem Rücklaufverhältnis von 1:1 fallen stündlich 1,8 l Rein-Acetylchlorid an.

Nach 204 Stunden wird der Versuch abgebrochen und eine Probe des gesammelten Destillats analysiert (Gewichts-%):

| | |
|---|---|
| Acetylchlorid | 99,56 |
| Acrylsäurechlorid | 0,21 |
| Essigsäureanhydrid | 0,23 |
| P-Gehalt | unter 1 ppm |

Während des Versuchs fielen insgesamt 81 l Sumpf im Umlaufverdampfer an.

### Patentanspruch

Verfahren zur Gewinnung reinen Acetylchlorids mit einem Phosphorgehalt unter 1 ppm, *dadurch gekennzeichnet,* daß man das rohe, Phosphorverbindungen enthaltende, Acetylchlorid in innige Berührung mit 1 bis 20, vorzugsweise 10 bis 15 Liter gasförmigem Chlor je Liter flüssiges, rohes Acetylchlorid bringt und anschließend in an sich bekannter Weise fraktioniert destilliert.

### Claim

Process for the production of pure acetyl chloride containing less than 1 ppm of phosphorus, wherein the crude acetyl chloride containing phosphorus compounds is intimately contacted with 1 to 20, preferably 10 to 15, litres of gaseous chlorine per litre of liquid crude acetyl chloride, and then subjected to customary fractional distillation.

### Revendication

Procédé pour l'obtention de chlorure d'acétyle pur ayant une teneur en phosphore de moins de 1 ppm, caractérisé en ce que l'on met le chlorure d'acétyle brut contenant des composés du phosphore en contact intime avec 1 à 20 litres, de préférence 10 à 15 litres, de chlore gazeux par litre de chlorure d'acétyle brut liquide et ensuite on soumet de manière connue à une distillation fractionnée.